# EUROPÄISCHE PATENTSCHRIFT

(11) **EP 0 616 998 B1**
(45) Veröffentlichungstag und Bekanntmachung des Hinweises auf die Patenterteilung: **01.05.1996**
(21) Anmeldenummer: 94810158.9
(22) Anmeldetag: 14.03.1994
(51) Int. Cl.: C07C 57/04, C07C 51/43, B01D 9/00

(54) **Verfahren und Vorrichtung zur Reinigung von Acrylsäure**
Process and plant for the purification of acrylic acid
Procédé et l'appareillage pour la purification d'acide acrylique

(30) Priorität: 26.03.1993 CH 922/93
(43) Veröffentlichungstag der Anmeldung: 28.09.1994
(73) Patentinhaber: Sulzer Chemtech AG, CH-8404 Winterthur (CH)
(72) Erfinder: Saxer, Kurt, CH-9470 Buchs (CH); Stadler, Ralph, CH-9470 Buchs (CH)
(74) Vertreter: Riederer, Conrad A., Dr.

(56) Entgegenhaltungen:
- DE-A- 1 620 756
- DE-A- 2 606 364
- FR-A- 1 079 676
- US-A- 2 868 830
- US-A- 2 885 431
- US-A- 3 050 952

## Beschreibung

Die Erfindung betrifft ein Verfahren und eine Vorrichtung zur Reinigung von Acrylsäure mittels fraktionierter Kristallisation.

Acrylsäure ist ein wichtiger Ausgangsstoff in der Kunststoffindustrie für Textilfasern, Oberflächenbeschichtungen, Adhäsive etc. Die jährlichen Produktionsmengen von Acrylsäure und Acrylsäureestern betragen jeweils mehrere hunderttausend Tonnen.

Von den verschiedenen, bekannten Herstellungsverfahren (Acrylnitril-Hydrolyse, katalytische Umsetzung von Acetylen mit Kohlenmonoxid und Wasser etc.) hat sich in jüngerer Zeit die einstufige und die zweistufige, heterogen-katalytische, Oxidation von Propen zu Acrylsäure via Acrolein durchgesetzt. Dank den differenzierten Reaktionsbedingungen und durch den Einsatz verschiedener Katalysatoren liefert die zweistufige Oxidation eine bessere Ausbeute als die einstufige. Diese beträgt bei der zweistufigen Oxidation etwas über 90%. Im Gegensatz dazu wird bei der einstufigen Oxidation lediglich eine Ausbeute von ungefähr 60% erzielt.

Die katalytische Oxidation von Propen zu Acrylsäure findet bei Temperaturen zwischen 200 und 300 Grad Celsius in der Gasphase statt. Die Acrylsäure wird anschliessend etwas abgekühlt und in einem Lösungsmittel, z.B. in Wasser oder in einem hochsiedenden Ester, aufgenommen. Die nachfolgende Reinigung, z.B. mittels Destillation, liefert dann Acrylsäure mit einem Reinheitsgrad von etwa 99%. Reine Acrylsäure schmilzt bei 13.5 Grad Celsius und siedet bei 141.6 Grad Celsius unter Normaldruck (760 Torr).

Da verschiedene Verunreingungen, wie z.B. Acrolein, Propionsäure etc., destillativ nicht vollständig entfernt werden können und die Acrylsäure ausserdem bei der Destillation gerne polymerisiert, wurden in der jüngeren Zeit zur Reinigung von Acrylsäure andere Reinigungsmethoden, z.B. die fraktionierte Kristallisation, vorgeschlagen. Limitierend für die Anwendung der fraktionierten Kristallisation für die Reinigung von in wässeriger Lösung vorliegender Acrylsäure ist jedoch, dass Acrylsäure mit Wasser ein Eutektikum bildet, welches bei 63% Volumengehalt Acrylsäure liegt. In der EP-0 002 612 wird deshalb vorgeschlagen, der wässerigen Lösung Salze zuzusetzen, um das Eutektikum aufzubrechen.

Die DE-OS-26 06 364 offenbart ein Verfahren zur Reinigung von Verbindungen mit einem Schmelzpunkt zwischen -50 bis +200°C mittels fraktionierter Kristallisation. Bei diesem Verfahren werden die Verbindungen in turbulenter Strömung durch eine stets gefüllte Kristallisationszone geschickt und so lange auf Kristallisationstemperatur belassen, bis eine Ausfrierrate von 70 bis 98% erreicht ist. Dieses Verfahren hat den Nachteil, dass die Trenneffizienz nachlässt, sobald der gewichtsmässige Verunreinigungsaneil ungefähr 20 bis 30% beträgt. Bei dieser Reinigungsmethode fällt daher eine grosse Rückstandsmenge an, die entsorgt oder anderweitig aufgearbeitet werden muss.

Bei der fraktionierten Kristallisation kann zwischen der statischen und der dynamischen Kristallisation unterschieden werden. Bei der dynamischen Kristallisation vom Typ Fallfilm oder volldurchströmtes Rohr wachsen die Kristalle an einer gekühlten Wand, entlang welcher eine Lösung oder eine Schmelze des Produktgemisches geleitet wird. Die Kristallisationswärme wird durch die entstehende Kristallschicht abgeführt, welche von aussen gekühlt wird. Dadurch ergibt sich eine hohe Kristallisationsgeschwindigkeit.

Bei den dynamischen Kristallisationsverfahren lässt die Trenneffizienz bei der Reinigung von Acrylsäure in Bereichen um die 70 - 80 Gewichtsprozente Produktgehalt nach. Der Grund dafür ist, dass die Acrylsäure bei einem Verunreinigungsanteil zwischen 20 und 30% in ungünstigen, z.B. dendritischen, Kristallformen auskristallisiert und die Kristallschichten schwammartig und weich sind. Diese Kristallschichten weisen im Verhältnis zum Volumen eine grosse Oberfläche auf, was grosse Benetzungsflächen und dadurch zusätzlich grossen Flüssigkeitsrückhalt bedeutet. Es fallen also bei der Reinigung mittels dynamischer Kristallisation relativ grosse Rückstandsmengen an. Diese Rückstände müssen den heutigen, strengen Umweltvorschriften entsprechend entsorgt werden. Eine Aufarbeitung der Rückstandsmengen ist meist wegen den darin vorkommenden, verschiedenen Verbindungen unwirtschaftlich.

Bei der statischen Kristallisation wird die zu reinigende Verbindung an in einen Behälter ragenden Kühlflächen auskristallisiert. Die fraktionierte, statische Kristallisation hat gegenüber der Fallfilmkristallisation den Nachteil, dass zur Erzielung einer gleich grossen Kapazität der statische Kristallisator grösser ausgelegt werden muss als ein entsprechender dynamischer Kristallisator, weil der Kristallisationsvorgang langsämer abläuft. Dementsprechend ist der Einsatz von statischer Kristallisation zur Reinigung von Acrylsäure im grosstechnischen Massstab nicht wirtschaftlich. Da ausserdem bekannterweise, wie schon oben erwähnt, die Kristallschichten umso schlechter, d.h. weicher und schwammartiger, werden, je höher der Verunreinigungsanteil ist, wurde der Einsatz von statischer Kristallisation zur Reinigung von Acrylsäure bisher nicht in Betracht gezogen. Bei der fraktionierten Suspensionskristalliation wird die Lösung resp. Schmelze bis unter die Sättigungstemperatur abgekühlt, sodass die Bildung von Kristallen initiiert wird. Die treibende Kraft der Kristallisation ist die Höhe der Uebersättigung der Lösung. Die anfallende Kristallisationswärme wird dabei durch die flüssige Phase abgeführt. Nachteilig bei der Suspensionskristallisation ist die gegenüber den vorgenannten Verfahren längere Kristallisationszeit, wodurch die Wirtschaftlichkeit dieses Verfahrens gemindert wird.

Aufgabe der vorliegenden Erfindung ist es, ein verbessertes, umweltverträglicheres und kostengünstigeres Verfahren zur Reinigung von Acrylsäure zur Verfügung zu stellen, welches die eingangs erwähnten Nachteile mindestens teilweise vermeidet und insbesondere die zu entsorgende Rückstandsmenge reduziert und die Ausbeute an reiner Acrylsäure erhöht.

Erfindungsgemäss wird dies durch ein Verfahren erreicht, bei welchem Acrylsäure durch eine Kombination von statischer und dynamischer Kristallisation in mehreren Stufen mittels Kristallisations-/Schmelzzyklen gereinigt wird, wobei der Rückstand der dynamischen Kristallisation durch die statische Kristallisation weiter gereinigt wird und die dabei gewonnene Acrylsäure wieder der dynamischen Kristallisation zugeführt wird und dass das Kristallisat der höchsten Stufe der dynamischen Kristallisation als Reinprodukt den Reinigungsprozess verlässt. Ueberraschenderweise wurde nämlich gefunden, dass bei der statischen Kristallisation bei entsprechender Prozessführung Kristallschichten erzeugt werden können, die auch bei einem hohen Verunreinigungsanteil von bis zu etwa 50 Gewichtsprozente genügend stabil ausgebildet werden, in einem Bereich also, in welchem die dynamische Kristallisation nicht mehr effizient arbeitet. Dadurch, dass die Kristallschichten wider Erwarten an den Kühlflächen des statischen Kristallisators haften bleiben, kann eine gute Trenneffizienz bei diesem hohen Verunreinigungsanteil erreicht werden. Somit kann zusätzlich Acrylsäure aus dem sonst zu entsorgenden oder mittels anderer Methoden aufzuarbeitenden Rückstand gewonnen werden. Die Rückstandsmengen des Reinigungsprozesses können somit deutlich vermindert werden.

Die Acrylsäure mit einem gewichtsmässigen Verunreinigungsanteil bis 20%, vorzugsweise bis etwa 10%, wird vorteilhaft einer Stufe der dynamischen Kristallisation zugeführt und in zwei oder mehreren Stufen im dynamischen Kristallisator gereinigt. In der untersten Stufe der dynamischen Kristallisation können die Verunreinigungen im Rückstand bis auf etwa 30 Gewichtsprozente, vorzugsweise auf 10% bis 25%, angereichert werden, wobei dieser Rückstand als Vorlage für die statische Kristallisation dient, in welcher der Rückstand in wenigstens einer Stufe gereinigt wird. Die Verunreinigungen im Rückstand, welcher den Reinigungsprozess verlässt, können dadurch bis auf ungefähr 50 Gewichtsprozente angereichert werden. Das mittels der statischen Kristallisation gewonnene Kristallisat der höchsten Stufe kann sodann wieder der dynamischen Kristallisation zugeführt werden. Dies ist ein rationelles und wirtschaftliches Verfahren. Von Bedeutung für das gute Funktionieren dieses Verfahrens ist jedoch, dass die Zusammensetzung der Vorlage für den statischen Kristallisator optimal ist. Es hat sich nämlich gezeigt, dass nicht nur der Verunreinigungsanteil von Bedeutung ist, sondern auch die qualitative Zusammensetzung der Vorlage. Es wurde gefunden, dass, wenn z.B. lineare, gesättigte Carbonsäuren wie Essigsäure und Propionsäure den Hauptanteil der Verunreinigungen ausmachen, die Kristallschichten schon bei einem niedrigen Verunreinigungsanteil weich und schwammig werden können. Wird der Verunreinigungsanteil in der niedrigsten Stufe der dynamischen Kristallisation auf 20-30% Gewichtsanteil aufkonzentriert, so liefert die statische Kristallisation keine befriedigenden Resultate mehr, weil die Haftung der Kristallschichten an den Kühlflächen nicht mehr ausreichend ist. Die dynamische Kristallisation muss deshalb, je nach der qualitativen Zusammensetzung der Verunreinigungen, beim Erreichen eines bestimmten Verunreinigungsanteils abgebrochen werden, obwohl noch mehr Acrylsäure aus dem Rückstand mittels dynamischer Kristallisation gewonnen werden könnte. Dadurch kann erreicht werden, dass sich eine erste stabile, gut haftende Kristallschicht mit günstigen Kristallformen an den Kühlflächen des statischen Kristallisators ausbildet. Diese bildet dann die Grundlage für die weiteren Kristallschichten.

Da nach dem Abschmelzen der Kristallschicht die Kühlflächen noch mit Schmelze befeuchtet sind, wird diese vorteilhaft ausgefroren, bevor der statische Kristallisator mit neuer, unreinerer Acrylsäure der nächstniedrigeren Stufe gefüllt wird. Durch diese Fahrweise kann wiederum eine gut haftende Kristallschicht gebildet werden, sodass eine weitere Stufe im statischen Kristallisator gefahren werden kann.

Das Verfahren hat ausserdem den Vorteil, dass auch Acrylsäure mit einem hohen, gewichtsmässigen Verunreinigungsanteil bis etwa 20% gereinigt werden kann. Ueblicherweise weist jedoch die heutzutage durch Oxidation von Propen hergestellte und anschliessend destillativ vorgereinigte Acrylsäure einen gewichtsmässigen Verunreinigungsanteil von weniger als 5% auf.

Zweckmässigerweise wird in der dynamischen Kristallisation der Rückstand und die partielle Schmelze einer bestimmten Stufe jeweils in einer niedrigeren Stufe der dynamischen Kristallisation einer folgenden Sequenz nochmals gereinigt. Die partielle Schmelze der niedrigsten Stufe der dynamischen Kristallisation wird vorteilhaft noch einmal in derselben Stufe gereinigt. Dies ist ein einfaches Verfahren, welches die Anreicherung der Verunreinigungen in der untersten Stufe bis auf etwa 30 Gewichtsprozente erlaubt.

Vorteilhaft laufen die statische und die dynamische Kristallisation gleichzeitig nebeneinander ab, d.h. während im statischen Kristallisator Chargen mit einem hohen Verunreinigungsanteil einem Kristallisations-/Schmelzzyklus unterworfen werden, werden im dynamischen Kristallisator Chargen mit einem geringeren Verunreinigungsanteil gereinigt. Dies ist zeitsparend und wirtschaftlich.

Vorteilhaft wird die partielle Schmelze der höchsten Stufe der statischen Kristallisation in zwei Teile unterteilt. Der erste Teil kann in der nächstniedrigeren Stufe und der zweite Teil in der selben Stufe einer nachfolgenden Sequenz gereinigt werden. Dadurch kann die Trenneffizienz der statischen Kristallisation erhöht werden, denn der zweite Teil der partiellen Schmelze ist meist bereits wesentlich reiner als der erste Teil.

Vorteilhaft wird in der statischen Kristallisation das Wärmeübertragungsmittel in der Kristallisationsphase während etwa einer Stunde bei der Starttemperatur von 0 °C bis -15 °C belassen und dann während 5 bis 6 Stunden auf die Endtemperatur von -30 °C bis -15 °C abgekühlt. Dies ist zweckmässig, denn durch das Konstanthalten der Temperatur des Wärmeübertragungsmittels in der Startphase können sich günstige Kristallformen bilden, die einen geringeren Verunreinigungsanteil aufweisen und darüberhinaus genügend stabil sind, sodass ein Abblättern der Kristallschichten von den Kühlflächen nicht stattfindet.

In der höchsten Stufe der dynamischen Kristallisation wird vorteilhaft lediglich das Kristallisat aus der vorhergehenden Stufe eingesetzt. Dadurch wird eine hohe Reinigungswirkung erreicht.

Zweckmässigerweise erfolgt die Reinigung von Acrylsäure mittels statischer Kristallisation in zwei Stufen. Dadurch kann der Verunreinigungsanteil im Rückstand bis auf etwa 50 Gewichtsprozente angereichert werden.

Die partielle Schmelze der ersten Stufe der statischen Kristallisation wird zweckmässigerweise in derselben Stufe und das entsprechende Kristallisat in der zweiten Stufe einer nachfolgenden Sequenz nochmals gereinigt.

Vorteilhaft wird die dynamische Kristallisation vom Typ Fallfilm oder volldurchströmtes Rohr eingesetzt. Diese beiden Methoden sind besonders effizient und schnell. Zur Erreichung eines Reinheitsgrades >99.9% wird die Acrylsäure vorteilhaft mittels dynamischer Kristallisation in 5 Stufen gereinigt. Zur Erreichung noch höherer Reinheitsanforderungen kann die Anzahl der Stufen entsprechend erhöht werden.

Zweckmässigerweise wird wenigstens einer weiteren Stufe der dynamischen Kristallisation kein neues Material zugesetzt. Dies kann dann geschehen, wenn z.B. ein relativ reines Einsatzprodukt eingesetzt wird. Dadurch kann ein Behälter eingespart werden, und die Anlagekosten reduzieren sich entsprechend.

Vorteilhaft erfolgt die Reinigung der Acrylsäure durch eine Kombination von Suspensionskristallisation und statischer Kristallisation in mehreren Stufen mittels Kristallisations-/Schmelzzyklen, wobei der Rückstand der Suspensionskristallisation durch die statische Kristallisation weiter gereinigt wird und die dabei gewonnene Acrylsäure wieder der Suspensionskristallisation zugeführt wird und dass das Kristallisat der höchsten Stufe der Suspensionkristallisation als Reinprodukt den Reinigungsprozess verlässt. Zweckmässigerweise wird Acrylsäure mit einem gewichtsmässigen Verunreinigungsanteil bis 20%, vorzugsweise bis etwa 10% einer Stufe der Suspensionskristallisation zugeführt und die Acrylsäure in einer oder mehreren Stufen gereinigt. In der untersten Stufe der Suspensionskristallisation werden die Verunreinigungen im Rückstand bis auf etwa 30 Gewichtsprozente, vorzugsweise auf 10% bis 25% angereichert, wobei dieser Rückstand als Vorlage für die statische Kristallisation dient. Die Prozessführung der statischen Kristallisation kann dabei, wie bereits oben beschrieben, erfolgen.

Zweckmässigerweise wird im dynamischen Kristallisationsverfahren das Wärmeübertragungsmittel während der Kristallisationsphase je nach Reinheit der Schmelze von +5 bis -5°C auf -10 bis -25°C abgekühlt und während der Schwitzphase auf bis zu ungefähr 18°C erwärmt. Dies erlaubt eine effiziente Prozessführung. Des weiteren ist vorteilhaft, wenn nach dem Abschmelzen der an den Kühloberflächen des statischen Kristallisators gebildeten Kristallschicht und Ablassen dieser Schmelze in einen Behälter der Kristallisator gekühlt und die an den Kühlflächen noch anhaftende Schmelze zu einer stabilen Kristallschicht auskristallisiert wird, bevor der Kristallisator mit einer neuen Charge gefüllt wird. Dadurch kann sich auf den Kühloberflächen eine stabile Kristallschicht mit einer günstigen Kristallstruktur ausbilden. Das erwähnte Ausfrieren der an den Kühlflächen noch anhaftenden Schmelze kann ebenfalls im dynamischen Kristallisationsverfahren angewendet werden.

Das Verfahren und eine Vorrichtung sollen nun mit Bezug auf die Figuren beschrieben werden. Es zeigt:
- Fig.1: eine Reinigungsvorrichtung mit einem statischen Kristallisator und einem dynamischen Kristallisator,
- Fig.2: eine Reinigungsvorrichtung mit einem statischen Kristallisator und einem Suspensionskristallisator,
- Fig.3: das Flussschema von zu reinigender Acrylsäure mit einem Reinheitsgrad von >99% während des Reinigungsprozesses,
- Fig.4: Flussschema von zu reinigender Acrylsäure mit einem Reinheitsgrad von ca. 97.8% während des Reinigungsprozesses.

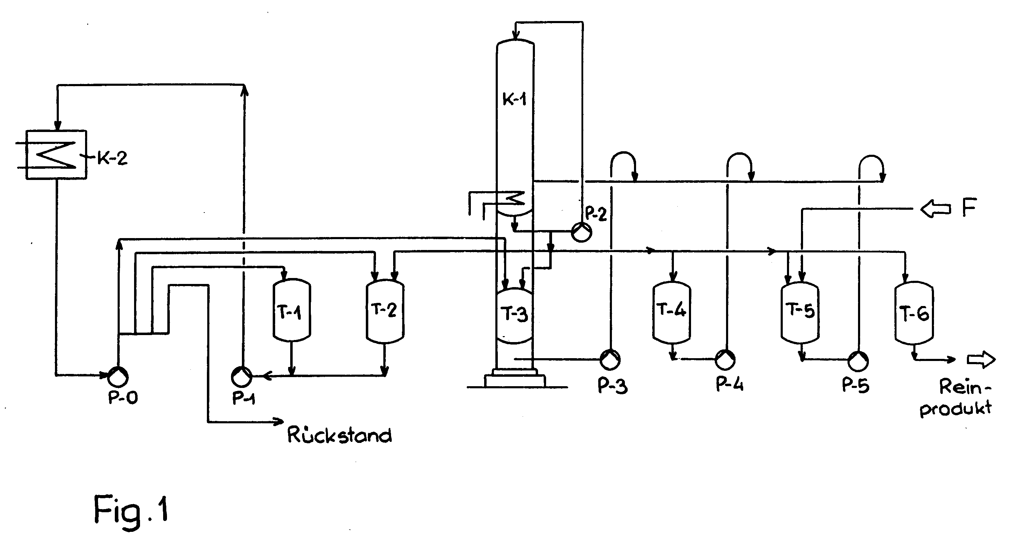

Fig.1 zeigt eine Reinigungsvorrichtung mit einem dynamischen Kristallisator K-1 und einem statischen Kristallisator K-2. Die Behälter T-1 bis T-5 dienen der Zwischenlagerung von Acrylsäure, welche nach einem Kristallisations-/Schmelzzyklus entweder als Rückstand R, partielle Schmelze S oder Kristallisat C in einen entsprechenden Behälter transferiert wird. Der Inhalt der Behälter T-1 bis T-5 wird nach der Zwischenlagerung wiederum einer entsprechenden Reinigungsstufe der statischen Kristallisation oder der dynamischen Kristallisation einer folgenden Sequenz zugeführt. Der Behälter T-6 ist ein Bufferbehälter und nimmt die gereinigte Acrylsäure auf.

Die Acrylsäure wird jeweils mittels einer Pumpe P-0 bis P-5 von den Behältern T-1 bis T-5 in die Kristallisatoren K-1 und K-2 gepumpt. Die Pumpe P-2 rezirkuliert die unten am Kristallisator K-1 austretende Schmelze während der Kristallisationsphase wieder zum Kopf des Kristallisators.

Der dynamische Kristallisator K-1 umfasst ein in einem Mantel untergebrachtes Rohrbündel, dessen Rohre mit einem Rieselfilm oder einer vollen Rohrströmung des zu reinigenden Einsatzproduktes F beschickbar sind. Aussen werden die Rohre des Rohrbündels mit einem Wärmeübertragungsmittel umspült, welches die bei der Kristallisation entstehende Wärme abführt. Die Temperatur des Wärmeübertragungsmittels befindet sich je nach Reinigungsstufe und Phase des Kristallisationsverfahrens in einem bestimmten Bereich unterhalb oder oberhalb des Erstarrungspunktes (Kristallisations- resp. Schmelzphase) der zu reinigenden Verbindung. Eine detaillierte Beschreibung eines dynamischen Kristallisators findet sich in der US- 3 621 664 an K. Saxer.

Der statische Kristallisator K-2 besteht aus einem Behälter mit einem Einlass und einem Auslass für das Einsatzprodukt F. In den Behälter tauchen Kühlflächen, die von einem Wärmeübertragungsmittel durchströmt werden können.

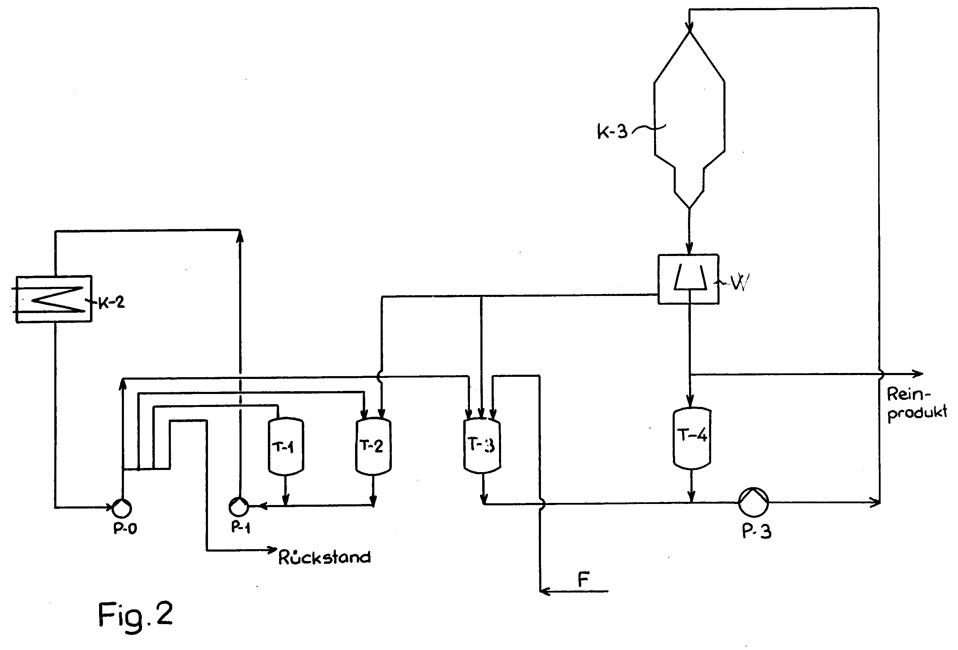

Fig. 2 zeigt schematisch eine Reinigungsvorrichtung mit einem Suspensionskristallisator K-3 und einem statischen Kristallisator K-2. Der Suspensionskristallisator K-3 besteht aus einem Behälter mit einem Eingang und einem Ausgang, welchem eine Vorrichtung V zum Abtrennen der Kristalle nachgeschaltet ist. Die Kristalle werden jeweils in den Behälter T-4 transferiert oder als Reinprodukt ausgeschieden, und die Mutterlauge kann je nach Stufe entweder in Behälter T-2 oder T-3 geleitet werden. Dem statischen Kristallisator K-2 können wie bei der Reinigungsvorrichtung gemäss Fig.1 zwei Behälter T-1 und T-2 zugeordnet sein. Je nach Verunreinigungsgehalt der eingesetzten Acrylsäure und gewünschter Reinheit kann die Vorrichtung in der Ausgestaltung zum obigen Beispiel differieren, z.B. indem diese mit einer grösseren oder kleineren Anzahl von Behältern ausgestattet ist.

### Das Verfahren

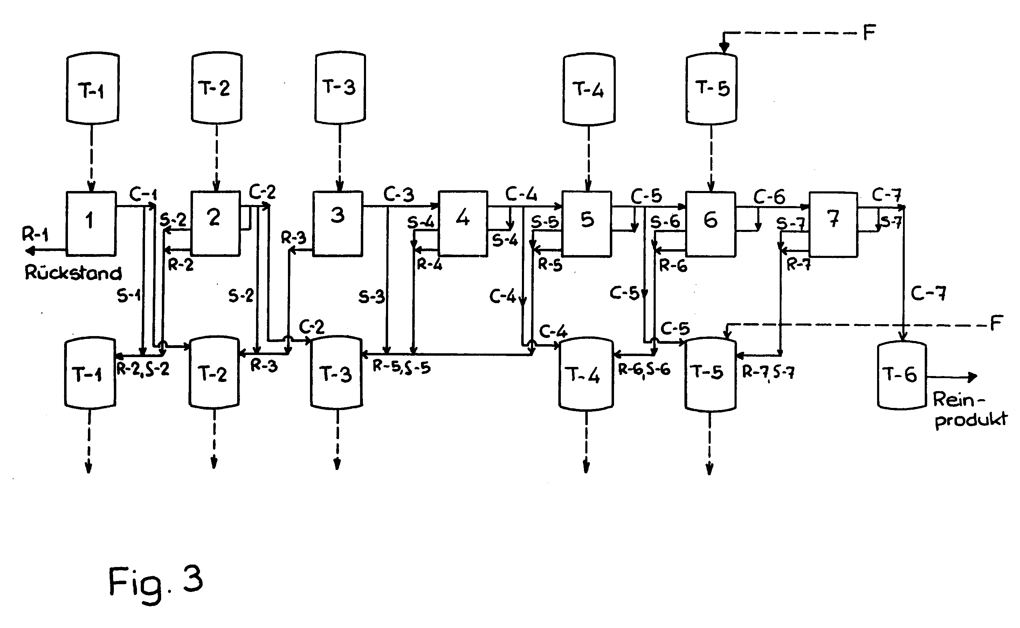

Nachfolgend soll nun die Reinigung von Acrylsäure nach dem erfindungsgemässen Verfahren anhand des Flussschemas der Fig.3 beschrieben werden. Die nachfolgenden Prozentangaben beziehen sich dabei jeweils auf Gewichtsprozente. Zur Erzielung eines Reinheitsgrades von >99.9% und eines gewichtsmässigen Verunreinigungsanteiles von etwa 40-50% im anfallenden Rückstand erfolgt die Reinigung der Acrylsäure mittels eines statischen und eines dynamischen Kristallisators in sieben Stufen. Die einzelnen Stufen entsprechen jeweils unterschiedlichen Reinheitsgraden und sind mit den Ziffern 1 bis 7 bezeichnet. Es ist dabei zu beachten, dass die Stufennummern 1 bis 7 sich nicht auf einen bestimmten, physischen Teil der Kristallisatoren, sondern auf eine Operation in der Prozessfolge beziehen.

Mittels der Stufen 1 und 2, bei welchen die höchsten Konzentrationen an Verunreinigungen vorliegen, erfolgt die Reinigung durch die statische Kristallisation, währenddem in den folgenden Stufen die Reinigung durch die dynamische Kristallisation erfolgt. Die nachgestellten Ziffern 1,2,3....bezeichnen den Rückstand R, die partielle Schmelze S und das Kristallisat C der jeweiligen Reinigungsstufen 1,2,3 etc. Je nach Reinheitsgrad des Einsatzproduktes F wird dasselbe einer Reinigungsstufe zugeführt, in welcher sich eine Mischung befindet, die eine ähnliche Zusammensetzung aufweist wie das Einsatzprodukt.

Im Fall der Reinigung eines bereits, beispielsweise destillativ, vorgereinigten Einsatzproduktes mit einem Reinheitsgrad >99% wird die flüssige Acrylsäure als Einsatzprodukt F in den Behälter T-5 geleitet, in welchem auch ein Teil des Kristallisats C-5 der fünften Stufe, der Rückstand R-7 und die partielle Schmelze S-7 gesammelt werden. Der Inhalt des Behälters T-5 dient als Vorlage für die sechste Reinigungsstufe. Dazu kommt ein Teil des geschmolzenen Kristallisats C-5 der fünften Stufe, falls eine vierte Stufe vor der fünften gefahren wird. Es ist zu beachten, dass in diesem Beispiel nicht immer alle Stufen hintereinander ablaufen, sondern dass z.B. die Stufen 5,6 und 7 öfters gefahren. Die Menge an unreinerer Acrylsäure wird z.B. im Behälter T-3 solange akkumuliert, bis eine volle Charge für den dynamischen Kristallisator erreicht ist. (Tab.3).

Die Pumpe P-5 pumpt den Inhalt des Behälters T-5 zum Kopf des Kristallisators K-1 und die Acrylsäure wird gleichmässig auf die einzelnen Rohre verteilt (Fig.1). An der Aussenseite der Rohre fliesst dabei gleichzeitig ein Wärmeübertragungsmittel. Auf der Innenseite der Rohre fliesst die Acrylsäure als Fallfilm oder als volle Rohrfüllung. Durch die Pumpe P-2 wird die unten am Kristallisator K-1 ausfliessende Acrylsäure wieder zum Kopf transportiert und auf die Rohre verteilt. Das Wärmeübertragungsmittel wird sodann rasch auf eine Temperatur abgekühlt, die je nach Stufe ungefähr 5 bis 20 Grad unterhalb des Erstarrungspunktes der Acrylsäure liegen kann. Die Acrylsäure beginnt in der Folge auf der Innenseite der Rohre als Kristallschicht auszukristallisieren. Die dabei anfallende Wärme wird durch die Kristallschicht und das an den Aussenwänden fliessende Wärmeübertragungsmittel abtransportiert. Das Wärmeübertragungsmittel wird in der Folge langsam und kontinuierlich weiter abgekühlt. Bei Erreichen einer bestimmten Kristallschichtdicke wird die Pumpe P-2 gestoppt und der Rückstand R-6 in den Behälter T-4 abgelassen.

Als nächster Schritt beginnt die sogenannte "Schwitzphase". Die Temperatur des Wärmeübertragungsmittels wird zu diesem Zweck soweit erhöht, bis die Kristallschicht partiell zu schmelzen beginnt. Dabei werden die auf der Oberfläche haftenden und in der Kristallschicht eingeschlossenen Verunreinigungen teilweise geschmolzen resp. herausgelöst. Die während der Schwitzphase der sechsten Stufe gebildete partielle Schmelze S-6 wird in den Behälter T-4 geleitet.

Das Kristallisat C-6 wird nun abgeschmolzen und unmittelbar in der siebten Reinigungsstufe eingesetzt. Im Unterschied zur sechsten Stufe wird in der siebten Reinigungsstufe also kein neues Material zugegeben. Man spricht deshalb auch von einer "Halbstufe". Es folgt ein weiterer Kristallisations-/Schmelzzyklus, wobei der Rückstand R-7 und die partielle Schmelze S-7 in den Behälter T-5 geleitet werden. Das Kristallisat C-7 verlässt den Reinigungskreislauf als Reinprodukt und wird im Behälter T-6 bis zum Abtransport zwischengelagert.

In der fünften Reinigungsstufe wird der Inhalt des Behälters T-4 in den Kristallisator K-1 gepumpt, in welchem sich eventuell noch das geschmolzene Kristallisat C-4 der vierten Stufe befindet. Der Inhalt des Behälters T-4 setzt sich zusammen aus dem Rückstand R-6, der partiellen Schmelze S-6 und eines Teils des Kristallisats C-4 der vorangegangenen Sequenz.

Der Rückstand R-5 und die partielle Schmelze S-5 der fünften Stufe werden in den Behälter T-3 geleitet. Ein Teil des geschmolzenen Kristallisats C-5 gelangt in den Behälter T-5, währenddem der andere Teil des Kristallisats C-5 zusammen mit dem Inhalt des Behälters T-5 der vorangegangenen Sequenz und frischem Einsatzprodukt F als Vorlage für die sechste Reinigungsstufe dient.

Es hat sich gezeigt, dass es vorteilhaft ist, die Kristallisate C-4,C-5 der Stufen vier und fünf jeweils aufzuteilen und teilweise in den Behälter, der die nächst höhere Stufe speist, einzuleiten. Dadurch wird ein Ausgleich der Konzentrationen und der Massenverhältnisse bewirkt, sodass der sechsten Stufe immer die gleichen Mengen an frischem Einsatzprodukt F zudosiert werden können, was die Automatisierung des Prozesses begünstigt. Ausserdem bleibt die Produktqualität der den Reinigungsprozess verlassenden Acrylsäure konstant.

Die Stufen 3 und 4 werden wie die Stufen 6 und 7 unmittelbar hintereinander gefahren, d.h. die vierte Stufe ist in diesem Beispiel wie Stufe 7 eine sogenannte Halbstufe, welcher kein neues Material zugeführt wird. In diesen Halbstufen wird also lediglich das Kristallisat der jeweils vorangegangenen Stufe eingesetzt. Als Vorlage für die dritte Stufe dient der Inhalt des Behälters T-3, in welchen die Rückstände R-4,R-5 und die partiellen Schmelzen S-3,S-4,S-5 und das Kristallisat C-2 der zweiten Stufe gesammelt werden. Der Rückstand R-3 der dritten Stufe wird in den Behälter T-2 geleitet. Das Ausmass der Aufkonzentrierung des Verunreinigungsanteils in der dritten Stufe hängt von der qualitativen und quantitativen Zusammensetzung desselben ab.

In der Stufe 2 wird die Acrylsäure vorzugsweise durch die statische Kristallisation gereinigt. Als Vorlage dient der Inhalt des Behälters T-2, welcher das geschmolzene Kristallisat C-1 sowie den Rückstand R-3 der dynamischen Kristallisation und der zweite Teil der partiellen Schmelze S-2 des statischen Kristallisators K-2 enthält. Die Trenneffizienz im statischen Kristallisator K-2 kann erhöht werden, wenn die partielle Schmelze S-2 in zwei Teile aufgeteilt wird. Der erste Teil, welcher eine grössere Konzentration an Verunreinigungen enthält, wird vorzugsweise in den Behälter T-1 der niedrigeren, ersten Stufe geleitet, der zweite Teil in den Behälter T-2 der Stufe 2. Der Inhalt des Behälters T-2 wird in den statischen Kristallisator K-2 gepumpt. Im statischen Kristallisator K-2 kristallisiert ein Teil der Acrylsäure an den Wärmetauscherplatten, welche in die Schmelze eintauchen. Nach einer bestimmten Zeit wird die nicht kristallisierte Schmelze als Rückstand R-2 in den Behälter T-1 transferiert. Der erste Teil der partiellen Schmelze S-2 wird ebenfalls dem Behälter T-1, der zweite Teil dem Behälter T-2 zugeführt.

Auf der ersten Stufe wird der Inhalt des Behälters T-1 aufgearbeitet. Der Rückstand R-1 verlässt dabei den Kreislauf, währenddem die partielle Schmelze S-1 in den Behälter T-1 und das Kristallisat in den Behälter T-2 geleitet wird.

Gemäss Flussdiagramm (Fig.3) ergibt sich also ein gegenläufiger Materialfluss, wobei der Reinheitsgrad der Acrylsäure von links nach rechts zunimmt. Die einzelnen Reinigungsstufen werden dabei in einer Sequenz immer von unten nach oben durchlaufen, d.h. vom weniger reinen zum reineren Produkt. Die Rückstände, die partiellen Schmelzen und eventuell abgeschmolzenes Kristallisat der einzelnen Stufen werden in den Behältern T-1 bis T-5 zwischengelagert und in einer nachfolgenden Sequenz wieder eingesetzt. Das Kristallisat C-3 bis C-6 verbleibt bis auf den Teil, der zwecks Ausgleich der Massenverhältnisse und Konzentrationen abgeschmolzen wird, immer im Kristallisator K-1 und wird in einer nächst höheren Stufe allein (Stufe 4 und 7) oder zusammen mit zwischengelagerter Acrylsäure aus einer oder mehreren vorangegangen Sequenzen nochmals einem Kristallisation/Schmelzzyklus unterworfen. Im statischen Kristallisator K-2 wird das Kristallisat C-1 und C-2 nach dem Schmelzen in die Behälter T-2 resp. T-3 transferiert.

Soll mit dem beschriebenen Verfahren ein Einsatzprodukt mit einem höheren Anteil an Verunreinigungen gereinigt werden, so wird das Einsatzprodukt in einen Behälter geleitet, dessen Inhalt eine Zusammensetzung aufweist, die etwa derjenigen des Einsatzproduktes entspricht.

Das Verfahren zur Reinigung von Acrylsäure bei Einsatz eines Suspensionskristallisators läuft im Prinzip gleich wie oben beschrieben ab. Bei der Verwendung der Reinigungsvorrichtung gemäss Fig.2 können jedoch eine geringere Anzahl von Stufen, z.B. vier Stufen, vorgesehen werden. Das Einsatzprodukt F wird in den Behälter T-3 geleitet und dann mittels der Pumpe P-3 in den Suspensionskristallisator K-3 befördert. Im Suspensionskristallisator K-3 beginnt dann die Acrylsäure auszukristallisieren (Stufe 3). Nach einer bestimmten Zeit wird der Vorgang abgebrochen und die Kristalle in der Trennvorrichtung V von der Mutterlauge getrennt. Die abfiltrierten Kristalle werden sodann geschmolzen, und die Schmelze wird im Behälter T-4 zwischengelagert. Die Mutterlauge wird in den Behälter T-2 geleitet, dessen Inhalt vom statischen Kristallisator z.B. in zwei Stufen gereinigt wird. Die Stufe 3 wird so oft gefahren, bis genügend Material für die vierte Stufe vorhanden ist. Das Kristallisat der vierten Stufe kann sodann den Reinigungsprozess verlassen, und der Rückstand dieser Stufe wird in den Behälter T-3 geleitet, in welchen ebenfalls die durch die statische Kristallisation gereinigte Acrylsäure transferiert wird.

Nachfolgend sollen nun Beispiele der Reinigung von Acrylsäure mittels des Verfahrens dargestellt werden.

### Beispiel 1:

Einsatzprodukt ist vorgereinigte Acrylsäure mit einem Produktgehalt von 99. 67%. Die Reinigung der Acrylsäure erfolgt gemäss Flussschema der Fig.3. Die gewichtsmässigen Anteile der Verunreinigungen im Einsatzprodukt, im gereinigten Produkt und im Rückstand können Tabelle 1 entnommen werden. Der Verunreinigungsanteil an der Schnittstelle zwischen dynamischer und statischer Kristallisation beträgt etwa 12 bis 15%.

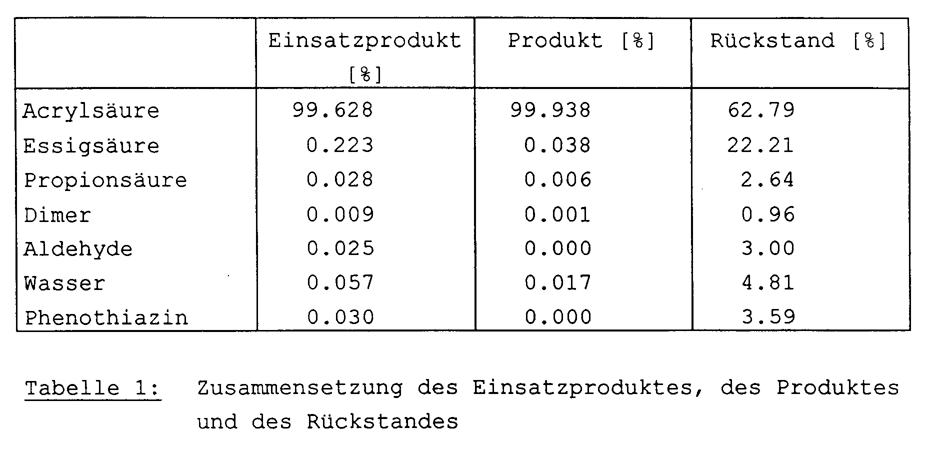

Aus der Tabelle 2 sind die Anfangstemperatur Tₛ und die Endtemperatur Tₑ des Wärmeübertragungsmittels während der Schwitz- und der Kristallisationsphase ersichtlich. In den angegebenen Zeitintervallen Δt wird das Wärmeübertragungsmittel von der Anfangstemperatur Tₛ gleichmässig auf die Endtemperatur Tₑ abgekühlt. Im statischen Kristallisator K-2 wird das Wärmeübertragungsmittel am Beginn der Abkühlungsphase vorteilhaft 1 Stunde auf der Anfangstemperatur belassen, um ein zu schnelles Auskristallisieren zu verhindern. Dies ist zweckmässig, weil damit die Bildung von ungünstigen Kristallformen, z.B. dendritische Kristalle, verhindert werden kann. Am Ende der Abkühlungsphase wird das Wärmeübertragungsmittel vorzugsweise zwischen 2 und 3 Stunden bei der Endtemperatur Tₑ belassen. Zum Abschmelzen des Kristallisats wird das Wärmeübertragungsmittel jeweils auf ungefähr 35 °C aufgewärmt. Die Schmelzphase dauert im statischen Kristallisator jeweils ca. eine Stunde und im Fallfilmkristallisator ca. 15 Minuten.

Der Erstarrungspunkt der Acrylsäure steigt von 5 °C in Stufe 3 auf 13 °C in Stufe 7 an. Die Ausbeute von Acrylsäure mit einem Reinheitsgrad >99.9% beträgt 99.5% bezogen auf das Einsatzprodukt. Der Rückstand enthält lediglich noch einen Anteil von 62.8% Acrylsäure.

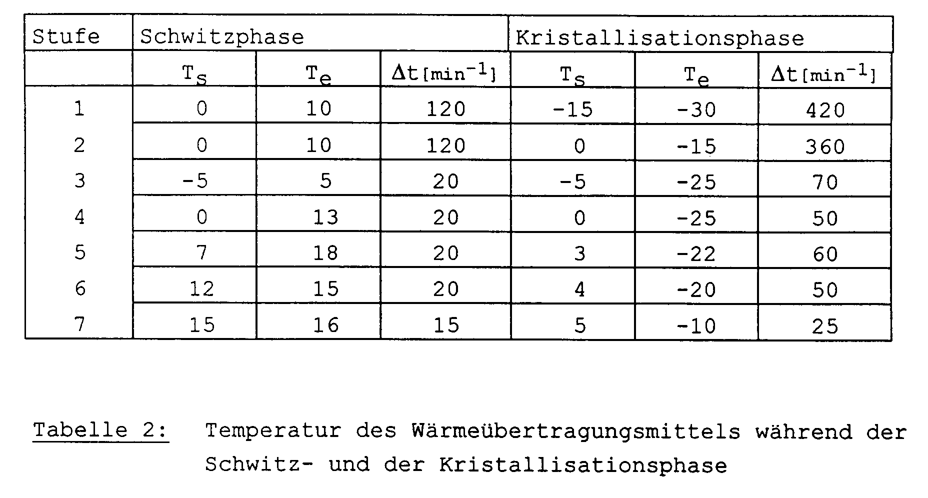

Zur Reinigung der Acrylsäure hat sich im dynamischen Kristallisator folgende Stufensequenz als vorteilhaft ergeben:

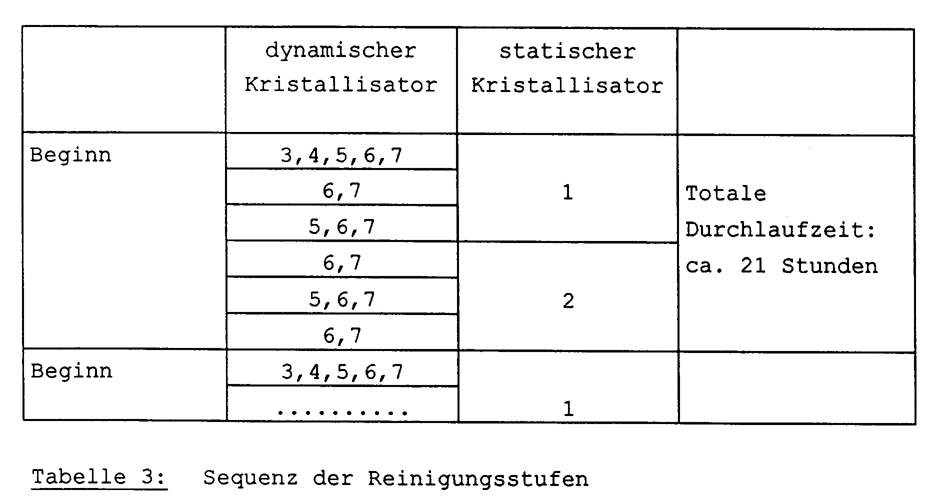

### Beispiel 2:

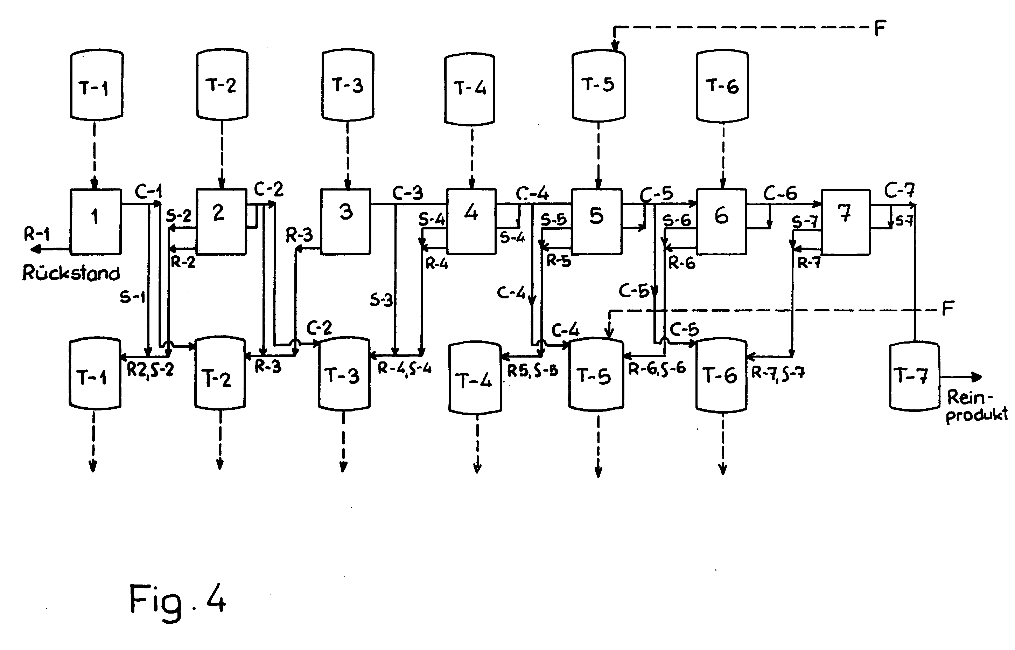

Einsatzprodukt ist vorgereinigte Acrylsäure mit einem Produktgehalt von 97.8%. Die Reinigung der Acrylsäure erfolgt gemäss Flussschema der Fig. 4. Die Unterschiede zum Verfahren gemäss Fig.3 sind wie folgt: Wegen der geringeren Reinheit des Einsatzproduktes wird dasselbe der Stufe 5 zugeleitet, deren Inhalt ungefähr die Zusammensetzung des Einsatzproduktes aufweist. Aufgrund der unterschiedlichen Ausgangskonzentration ergeben sich unterschiedliche Massenverhältnisse, sodass die Stufe 4 als Vollstufe gefahren werden kann, d.h. es wird im Unterschied zum 1. Beispiel ein zusätzlicher Behälter T-4 für die vierte Stufe eingesetzt.

Die gewichtsmässigen Anteile der Verunreinigungen im Einsatzprodukt, im gereinigten Produkt und im Rückstand können Tabelle 1 entnommen werden.

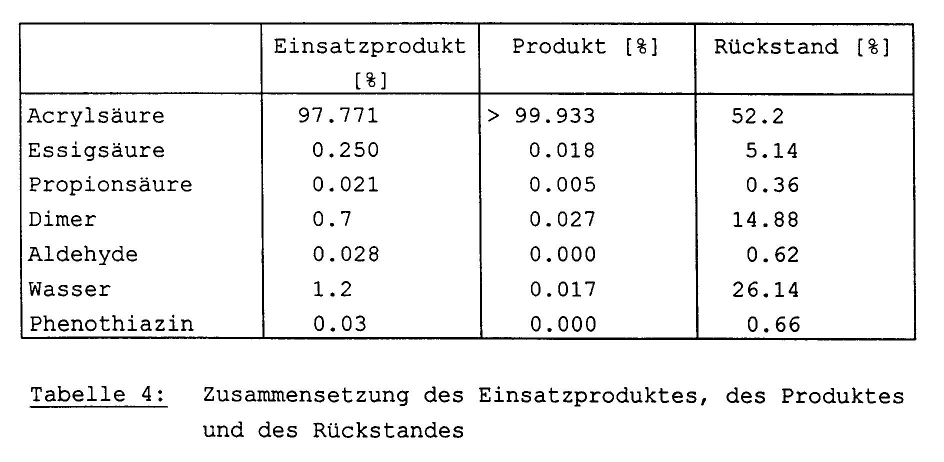

Die Anfangstemperaturen Tₛ und die Endtemperaturen Tₑ des Wärmeübertragungsmittels während der Schwitz- und der Kristallisationsphase sowie die einzelnen Zeitintervalle Δt, während welchen das Wärmeübertragungsmittel von der Anfangstemperatur Tₛ auf die Endtemperatur Tₑ abgekühlt wird, stimmen im wesentlichen mit denjenigen des Beispiels 1 überein.

Zur Reinigung der Acrylsäure hat sich im dynamischen Kristallisator folgende Stufensequenz als vorteilhaft ergeben:

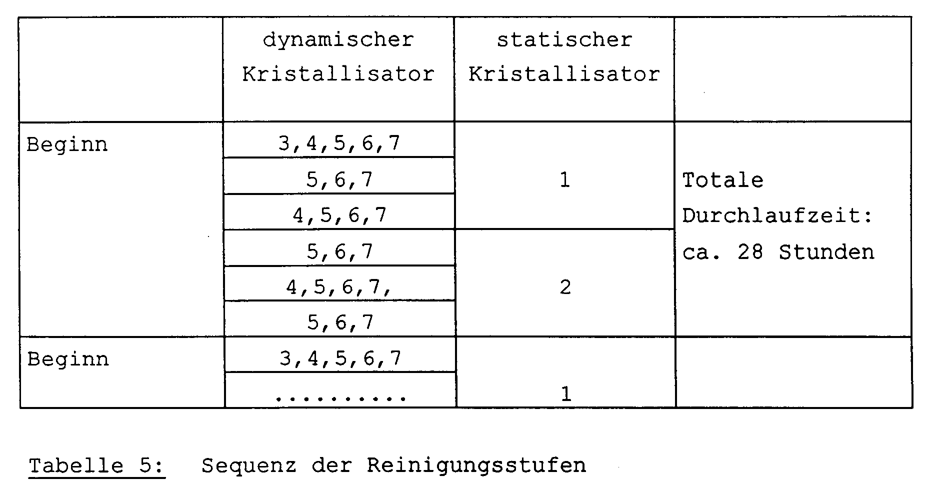

## Patentansprüche

1. Verfahren zur Reinigung von Acrylsäure mittels fraktionierter Kristallisation, dadurch gekennzeichnet, dass Acrylsäure durch eine Kombination von dynamischer und statischer Kristallisation in mehreren Stufen mittels Kristallisations-/Schmelzzyklen gereinigt wird, wobei der Rückstand der dynamischen Kristallisation durch die statische Kristallisation weiter gereinigt wird und die dabei gewonnene Acrylsäure wieder der dynamischen Kristallisation zugeführt wird und dass das Kristallisat der höchsten Stufe der dynamischen Kristallisation als Reinprodukt den Reinigungsprozess verlässt.

2. Verfahren nach Anspruch 1, dadurch gekennzeichnet, dass Acrylsäure mit einem gewichtsmässigen Verunreinigungsanteil bis etwa 20%, vorzugsweise bis etwa 10% einer Stufe der dynamischen Kristallisation zugeführt und die Acrylsäure in zwei oder mehreren Stufen im dynamischen Kristallisator gereinigt wird, wobei in der untersten Stufe der dynamischen Kristallisation die Verunreinigungen im Rückstand bis auf etwa 30%, vorzugsweise auf 10% bis 25%, angereichert werden, wobei dieser Rückstand als Vorlage für die statische Kristallisation dient, in welcher der Rückstand in wenigstens einer Stufe gereinigt wird, wodurch die Verunreinigungen im Rückstand, welcher den Reinigungsprozess verlässt, bis auf ungefähr 50 Gewichtsprozente angereichert werden können, und das gewonnene Kristallisat der höchsten Stufe der statischen Kristallisation wieder der dynamischen Kristallisation zugeführt wird.

3. Verfahren nach Anspruch 1 oder 2, dadurch gekennzeichnet, dass in der dynamischen Kristallisation der Rückstand und die partielle Schmelze einer bestimmten Stufe jeweils in einer niedrigeren Stufe der dynamischen Kristallisation einer folgenden Sequenz nochmals gereinigt werden und die partielle Schmelze der niedrigsten Stufe der dynamischen Kristallisation in derselben Stufe noch einmal gereinigt wird.

4. Verfahren nach einem der Ansprüche 1 bis 3, dadurch gekennzeichnet, dass die statische und die dynamische Kristallisation gleichzeitig nebeneinander ablaufen.

5. Verfahren nach einem der Ansprüche 1 bis 4, dadurch gekennzeichnet, dass die partielle Schmelze der höchsten Stufe der statischen Kristallisation in zwei Teile unterteilt wird, wobei der erste Teil in der nächstniedrigeren Stufe und der zweite Teil in derselben Stufe einer nachfolgenden Sequenz gereinigt werden.

6. Verfahren nach einem der Ansprüche 1 bis 5, dadurch gekennzeichnet, dass in der statischen Kristallisation das Wärmeübertragungsmittel in der Kristallisationsphase während etwa einer Stunde bei der Starttemperatur von 0 Grad bis -15 Grad belassen und dann während 5 bis 6 Stunden auf die Endtemperatur von -30 Grad bis -15 Grad Celsius abgekühlt wird.

7. Verfahren nach einem der Ansprüche 1 bis 6, dadurch gekennzeichnet, dass in der höchsten Stufe der dynamischen Kristallisation lediglich das Kristallisat aus der vorhergehenden Stufe eingesetzt wird.

8. Verfahren nach einem der Ansprüche 1 bis 7, dadurch gekennzeichnet, dass die Reinigung von Acrylsäure mittels statischer Kristallisation in zwei Stufen erfolgt.

9. Verfahren nach Anspruch 8, dadurch gekennzeichnet, dass die partielle Schmelze der ersten Stufe in der ersten Stufe und das entsprechende Kristallisat in der zweiten Stufe einer nachfolgenden Sequenz nochmals gereinigt wird.

10. Verfahren nach einem der Ansprüche 1 bis 9, dadurch gekennzeichnet, dass die dynamische Kristallisation vom Typ Fallfilm oder volldurchströmtes Rohr eingesetzt wird.

11. Verfahren nach Anspruch 10, dadurch gekennzeichnet, dass zur Erreichung eines Reinheitsgrades >99.9% die Acrylsäure mittels dynamischer Kristallisation in 5 Stufen gereinigt wird.

12. Verfahren nach einem der Ansprüche 7 bis 11, dadurch gekennzeichnet, dass wenigstens einer weiteren Stufe der dynamischen Kristallisation kein neues Material zugesetzt wird, d.h. diese als Halbstufe gefahren wird.

13. Verfahren zur Reinigung von Acrylsäure mittels fraktionierter Kristallisation, dadurch gekennzeichnet, dass Acrylsäure durch eine Kombination von Suspensionskristallisation und statischer Kristallisation in mehreren Stufen mittels Kristallisations-/Schmelzzyklen gereinigt wird, wobei der Rückstand der Suspensionskristallisation durch die statische Kristallisation weiter gereinigt wird und die dabei gewonnene Acrylsäure wieder der Suspensionskristallisation zugeführt wird und dass das Kristallisat der höchsten Stufe der Suspensionkristallisation als Reinprodukt den Reinigungsprozess verlässt.

14. Verfahren nach Anspruch 13, dadurch gekennzeichnet, dass Acrylsäure mit einem gewichstmässigen Verunreinigungsanteil bis 20%, vorzugsweise bis etwa 10% einer Stufe der Suspensionskristallisation zugeführt und die Acrylsäure in einer oder mehreren Stufen gereinigt wird, wobei in der untersten Stufe der Suspensionskristallisation die Verunreinigungen im Rückstand bis auf etwa 30%, vorzugsweise 10 bis 25%, angereichert werden, wobei dieser Rückstand als Vorlage für die statische Kristallisation dient, in welcher der Rückstand in wenigstens einer Stufe gereinigt wird, wodurch die Verunreinigungen im Rückstand, welcher den Reinigungsprozess verlässt, bis auf ungefähr 50 Gewichtsprozente angereichert werden können, und das gewonnene Kristallisat der höchsten Stufe der statischen Kristallisation wieder der dynamischen Kristallisation zugeführt wird.

15. Verfahren nach einem der Ansprüche 1 bis 14, dadurch gekennzeichnet, dass im dynamischen Kristallisationsverfahren das Wärmeübertragungsmittel während der Kristallisationsphase je nach Reinheit der Schmelze von etwa +5 bis -5°C auf etwa -10 bis -25°C abgekühlt und während der Schwitzphase auf bis zu ungefähr 18°C erwärmt wird.

16. Verfahren nach einem der Ansprüche 1 bis 15, dadurch gekennzeichnet, dass nach dem Abschmelzen der an den Kühloberflächen des statischen Kristallisators gebildeten Kristallschicht und Ablassen dieser Schmelze in einen Behälter der Kristallisator gekühlt und die an den Kühlflächen noch anhaftende Schmelze zu einer stabilen Kristallschicht auskristallisiert wird, bevor der Kristallisator mit einer neuen Charge gefüllt wird.

17. Vorrichtung zur Reinigung von Acrylsäure mittels fraktionierter Kristallisation, gekennzeichnet durch wenigstens einen statischen Kristallisator und einen dynamischen Kristallisator, welche in Serie geschaltet sind, wobei der statische Kristallisator zur Reinigung des Rückstandes der dynamischen Kristallisation vorgesehen ist, mehrere Behälter zur Zwischenlagerung von Acrylsäurefraktionen, sowie Pumpen und Röhren für den Transport der Acrylsäure von den Behältern zu den Kristallisatoren und umgekehrt.

18. Vorrichtung nach Anspruch 17, dadurch gekennzeichnet, dass ein statischer Kristallisator, ein dynamischer Kristallisator vom Typ Fallfilm oder volldurchströmtes Rohr und fünf oder sechs Behälter zur Zwischenlagerung der Acrylsäure vorgesehen sind.

19. Vorrichtung zur Reinigung von Acrylsäure mittels fraktionierter Kristallisation, gekennzeichnet durch wenigstens einen statischen Kristallisator und einen Suspensionskristallisator, welche in Serie geschaltet sind, wobei der statische Kristallisator zur Reinigung des Rückstandes der Suspensionkristallisation vorgesehen ist, mehrere Behälter zur Zwischenlagerung von Acrylsäurefraktionen, sowie Pumpen und Röhren für den Transport der Acrylsäure von den Behältern zu den Kristallisatoren und umgekehrt.

20. Vorrichtung nach Anspruch 19, dadurch gekennzeichnet, dass ein statischer Kristallisator, ein Suspensionskristallisator und wenigstens vier Behälter zur Zwischenlagerung der Acrylsäure vorgesehen sind.

## Claims

1. A method of purifying acrylic acid by fractional crystallisation, characterised in that acrylic acid is purified in a number of stages in crystallisation/melting cycles by a combination of dynamic and static crystallisation, the residue from dynamic crystallisation being further purified by static crystallisation and the resulting acrylic acid being returned to dynamic crystallisation, the crystallised material from the highest stage of dynamic crystallisation leaving the purification process in the form of pure product.

2. A method according to claim 1, characterised in that acrylic acid containing up to 20%, preferably up to 10% by weight of impurities is supplied to a dynamic crystallisation stage and the acrylic acid is purified in two or more stages in a dynamic crystalliser, the impurities in the residue are concentrated in the lowest stage of dynamic crystallisation to about 30%, preferably 10% to 25%, the residue serving as a feed for static crystallisation, in which the residue is purified in at least one stage, whereby the impurities in the residue leaving the purification process can be concentrated to about 50% by weight, and the crystallised material obtained from the highest stage of static crystallisation is returned to dynamic crystallisation.

3. A method according to claim 1 or 2, characterised in that the residue in dynamic crystallisation and the partial melt from a given stage are each additionally purified in a lower stage of dynamic crystallisation in a subsequent sequence, and the partial melt from the lowest stage of dynamic crystallisation is re-purified in the same stage.

4. A method according to any of claims 1 to 3, characterised in that the static and dynamic crystallisation occur simultaneously side by side.

5. A method according to any of claims 1 to 4, characterised in that the partial melt from the highest stage of static crystallisation is divided into two parts, the first part being purified in the next lower stage and the second part in the same stage of a subsequent sequence.

6. A method according to any of claims 1 to 5, characterised in that in the static crystallisation, the heat transfer medium in the crystallisation phase is left at the starting temperature of 0 to -15°C for about 1 hour and then cooled to the final temperature of -30 to -15°C for 5 to 6 hours.

7. A method according to any of claims 1 to 6, characterised in that in the highest stage of dynamic crystallisation, only the crystallised material from the previous stage is used.

8. A method according to any of claims 1 to 7, characterised in that the acrylic acid is purified by static crystallisation in two stages.

9. A method according to claim 8, characterised in that the partial melt from the first stage is purified in the first stage of a subsequent sequence and the corresponding crystallised material is purified in the second stage thereof.

10. A method according to any of claims 1 to 9, characterised in that the dynamic crystallisation is of the falling-film or fully flowed-through tube type.

11. A method according to claim 10, characterised in that in order to obtain purity above 99.9%, the acrylic acid is purified by dynamic crystallisation in five stages.

12. A method according to any of claims 7 to 11, characterised in that no new material is added in at least one additional stage of dynamic crystallisation, i.e. the stage is operated as a half-stage.

13. A method of purifying acrylic acid by fractional crystallisation, characterised in that acrylic acid is purified by a combination of suspension crystallisation and static crystallisation in a number of stages in crystallisation/melting cycles, the residue from suspension crystallisation being further purified by static crystallisation and the resulting acrylic acid being returned to suspension crystallisation, the crystallised material from the highest stage of suspension crystallisation leaving the purification process in the form of pure product.

14. A method according to claim 13, characterised in that acrylic acid containing up to 20%, preferably up to about 10% by weight of impurities is supplied to a suspension crystallisation stage and the acrylic acid is purified in one or more stages, the impurities in the residue are concentrated in the lowest stage of suspension crystallisation to about 30%, preferably 10 to 25%, the residue serving as a feed for static crystallisation, in which the residue is purified in at least one stage whereby the impurities in the residue leaving the purification process can be concentrated to about 50% by weight and the crystallised material obtained from the highest stage of static crystallisation is returned to dynamic crystallisation.

15. A method according to any of claims 1 to 14, characterised in that in the dynamic crystallisation process the heat transfer medium, depending on the purity of the melt, is cooled from about 5 to -5°C to about -10 to -25°C during the crystallisation phase and heated to about 18°C during the sweating phase.

16. A method according to any of claims 1 to 15, characterised in that after the crystal layer formed on the cooling surfaces of the static crystalliser has melted and after the melt has been discharged into a tank, the crystalliser is cooled and the melt still adhering to the cooling surfaces is crystallised out to form a stable crystal layer before the crystalliser is filled with a new batch.

17. Apparatus for purifying acrylic acid by fractional crystallisation, characterised by at least one static crystalliser and one dynamic crystalliser connected in series, the static crystalliser being provided for purifying the residue from dynamic crystallisation, and also characterised by tanks for intermediate storage of acrylic acid fractions, and pumps and pipes for conveying the acrylic acid from the tanks to the crystallisers and vice versa.

18. Apparatus according to claim 17, characterised in that a static crystalliser, a dynamic crystalliser of the falling-film or fully flowed-through tube type and five or six tanks for intermediate storage of acrylic acid are provided.

19. Apparatus for purifying acrylic acid by fractional crystallisation, characterised by at least one static crystalliser and one suspension crystalliser connected in series, the static crystalliser being provided for purifying the residue from suspension crystallisation, the device also comprising tanks for intermediate storage of acrylic acid fractions, and pumps and pipes for conveying the acrylic acid from the tanks to the crystallisers and vice versa.

20. Apparatus according to claim 19, characterised in that a static crystalliser, a suspension crystalliser and at least four tanks for intermediate storage of acrylic acid are provided.

## Revendications

1. Procédé de purification d'acide acrylique par cristallisation fractionnée, caractérisé en ce que de l'acide acrylique est purifié par une combinaison de cristallisations dynamique et statique en plusieurs échelons au moyen de cycles de cristallisation/fusion, le résidu de la cristallisation dynamique étant purifié à un degré plus élevé par la cristallisation statique et l'acide acrylique ainsi obtenu étant renvoyé dans la cristallisation dynamique, et en ce que le produit de cristallisation du plus haut échelon de la cristallisation dynamique quitte le traitement de purification en tant que produit pur.

2. Procédé selon la revendication 1, caractérisé en ce que de l'acide acrylique contenant une proportion en poids d'impuretés d'environ 20% au maximum, de préférence d'environ 10% au maximum, est dirigé vers un échelon de la cristallisation dynamique et l'acide acrylique est purifié en deux ou plusieurs échelons dans le cristallisateur dynamique, les impuretés dans le résidu étant enrichies jusqu'à 30% environ, de préférence à 10-25% dans le plus bas échelon de la cristallisation dynamique, ce résidu servant de charge d'alimentation pour la cristallisation statique, dans laquelle le résidu est purifié en un échelon au moins, d'où il résulte que les impuretés dans le résidu qui quitte le traitement de purification peuvent être enrichies jusqu'a 50% en poids environ, et le produit de cristallisation obtenu dans le plus haut échelon de la cristallisation statique est renvoyé dans la cristallisation dynamique.

3. Procédé selon la revendication 1 ou 2, caractérisé en ce que, dans la cristallisation dynamique, le résidu et la masse fondue partielle d'un échelon déterminé sont chaque fois purifiés de nouveau dans un échelon inférieur de la cristallisation dynamique d'une séquence subséquente et la masse fondue partielle du plus bas échelon de la cristallisation dynamique est purifiée une fois encore dans le même échelon.

4. Procédé selon l'une quelconque des revendications 1 à 3, caractérisé en ce que la cristallisation statique et la cristallisation dynamique se déroulent côte à côte simultanément.

5. Procédé selon l'une quelconque des revendications 1 à 4, caractérisé en ce que la masse fondue partielle du plus haut échelon de la cristallisation statique est divisée en deux parties, la première partie étant purifiée dans l'échelon immédiatement inférieur d'une séquence subséquente et la seconde partie étant purifiée dans le même échelon de cette séquence.

6. Procédé selon l'une quelconque des revendications 1 à 5, caractérisé en ce que, dans la cristallisation statique, le caloporteur dans la phase de cristallisation est laissé pendant une heure environ à la température initiale de 0° à -15°C, puis est refroidi, pendant 5 à 6 h, à la température finale de -30° à -15°C.

7. Procédé selon l'une quelconque des revendications 1 à 6, caractérisé en ce qu'il est seulement introduit, dans le plus haut échelon de la cristallisation dynamique, le produit de cristallisation provenant de l'échelon précédent.

8. Procédé selon l'une quelconque des revendications 1 à 7, caractérisé en ce que la purification d'acide acrylique au moyen de cristallisation statique est effectuée en deux échelons.

9. Procédé selon la revendication 8, caractérisé en ce que la masse fondue partielle du premier échelon est purifiée de nouveau dans le premier échelon d'une séquence subséquente et le produit de cristallisation correspondant l'est dans le second échelon de cette séquence.

10. Procédé selon l'une quelconque des revendications 1 à 9, caractérisé en ce qu'il est fait usage de la cristallisation dynamique du type à film tombant ou à tube complètement parcouru par le courant.

11. Procédé selon la revendication 10, caractérisé en ce que, pour atteindre un degré de pureté >99,9%, l'acide acrylique est purifié par cristallisation dynamique en 5 échelons.

12. Procédé selon l'une quelconque des revendications 7 à 11, caractérisé en ce que, dans au moins un échelon supplémentaire de la cristallisation dynamique, il n'est pas ajouté de nouvelle substance, c'est-à-dire que cet échelon est conduit comme un demi-échelon.

13. Procédé de purification d'acide acrylique par cristallisation fractionnée, caractérisé en ce que de l'acide acrylique est purifié par une combinaison de cristallisation en suspension et de cristallisation statique en plusieurs échelons au moyen de cycles de cristallisation/fusion, le résidu de la cristallisation en suspension étant purifié à un degré plus élevé par la cristallisation statique et l'acide acrylique ainsi obtenu étant renvoyé dans la cristallisation en suspension, et en ce que le produit de cristallisation du plus haut échelon de la cristallisation en suspension quitte le traitement de purification en tant que produit pur.

14. Procédé selon la revendication 13, caractérisé en ce que de l'acide acrylique contenant une proportion en poids d'impuretés de 20% au maximum, de préférence d'environ 10% au maximum, est dirigé vers un échelon de la cristallisation en suspension et l'acide acrylique est purifié en un ou plusieurs échelons, les impuretés dans le résidu étant enrichies jusqu'à 30% environ, de préférence à 10-25% dans le plus bas échelon de la cristallisation en suspension, ce résidu servant de charge d'alimentation pour la cristallisation statique, dans laquelle le résidu est purifié en un échelon au moins, d'où il résulte que les impuretés dans le résidu qui quitte le traitement de purification peuvent être enrichies jusqu'à 50% en poids environ, et le produit de cristallisation obtenu dans le plus haut échelon de la cristallisation statique est renvoyé dans la cristallisation dynamique.

15. Procédé selon l'une quelconque des revendications 1 à 14, caractérisé en ce que, dans le procédé de cristallisation dynamique, le caloporteur est refroidi pendant la phase de cristallisation, selon la pureté de la masse fondue, d'une valeur de +5 à -5°C environ à une valeur de -10 à -25°C environ et est réchauffé, pendant la phase de ressuage, à une température pouvant atteindre 18°C environ.

16. Procédé selon l'une quelconque des revendications 1 à 15, caractérisé en ce qu'après que la couche de cristaux formée sur les surfaces de refroidissement du cristallisateur statique a été fondue et que cette masse fondue à été vidée dans un récipient, le cristallisateur est refroidi et la masse fondue qui adhère encore aux surfaces de refroidissement est séparée par cristallisation en une couche stable de cristaux, avant que le cristallisateur ne soit rempli d'une nouvelle charge.

17. Dispositif pour la purification d'acide acrylique par cristallisation fractionnée, caractérisé par au moins un cristallisateur statique et un cristallisateur dynamique qui sont montés en série, le cristallisateur statique étant prévu pour la purification du résidu de la cristallisation dynamique, par plusieurs réservoirs pour l'entreposage de fractions d'acide acrylique, ainsi que par des pompes et des canalisations pour le transport de l'acide acrylique des réservoirs aux cristallisateurs et vice versa.

18. Dispositif selon la revendication 17, caractérisé en ce qu'il est prévu un cristallisateur statique, un cristallisateur dynamique du type à film tombant ou à tube complètement parcouru par le courant et cinq ou six réservoirs pour l'entreposage de l'acide acrylique.

19. Dispositif pour la purification d'acide acrylique par cristallisation fractionnée, caractérisé par au moins un cristallisateur statique et un cristallisateur en suspension qui sont montés en série, le cristallisateur statique étant prévu pour la purification du résidu de la cristallisation en suspension, par plusieurs réservoirs pour l'entreposage de fractions d'acide acrylique, ainsi que par des pompes et des canalisations pour le transport de l'acide acrylique des réservoirs aux cristallisateurs et vice versa.

20. Dispositif selon la revendication 19, caractérisé en ce qu'il est prévu un cristallisateur statique, un cristallisateur en suspension et au moins quatre réservoirs pour l'entreposage de l'acide acrylique.
